# EUROPEAN PATENT SPECIFICATION

(11) **EP 2 124 054 B1**
(45) Date of publication and mention of the grant of the patent: **01.11.2017**
(21) Application number: 09251218.5
(22) Date of filing: 29.04.2009
(51) Int. Cl.: G01N 33/53, G01N 35/00

(54) **Immunodiagnostic test apparatus having at least one imager to provide advance agglutination evaluations during centrifugation cycle**
Immunodiagnostiktestvorrichtung mit mindestens einem Bildgerät, um Vorabagglutinationsschätzungen während eines Zentrifugationszyklus bereitzustellen
Appareil de tests d'immunodiagnostic ayant au moins un imageur pour fournir des évaluations d'agglutination préalables pendant le cycle de centrifugation

(30) Priority: 30.04.2008 US 112343
(43) Date of publication of application: 25.11.2009
(73) Proprietor: Ortho-Clinical Diagnostics, Inc., Rochester, NY 14626-5101 (US)
(72) Inventor: Jakubowicz, Raymond F., Rush, NY 14543 (US); Moran, Donald J., Jr., Rochester, NY 14618 (US); Dee, Michael L., Livonia, NY 14487 (US); Chiapperi, Joseph M., Rochester, NY 14612 (US); Sawczuk, Mark, Rochester, NY 14626 (US)
(74) Representative: Goodfellow, Hugh Robin

(56) References cited:
- EP-A2- 1 450 159
- EP-A2- 1 526 370
- US-A- 4 297 104
- US-A- 5 578 269
- DATABASE WPI Week 199125 Thomson Scientific, London, GB; AN 1991-181987 XP002540066 -& JP 03 110468 A (ANALYTICAL INSTR KK) 10 May 1991 (1991-05-10)

## Description

### FIELD OF THE INVENTION

The application relates to the field of immunodiagnostic testing and more particularly to an automated testing apparatus having at least one imager disposed in relative proximity to a centrifuge used for supporting at least one test sample. The at least one imager is configured to provide images of test samples in order to enable enhanced processing in advance of a fully completed centrifugation cycle.

### BACKGROUND OF THE INVENTION

Immunological agglutination reactions are used for identifying various kinds of blood types as well as for detecting various kinds of antibodies and antigens in blood samples and other aqueous solutions. In such procedures, a sample of red blood cells is mixed with serum or plasma in either test tubes or microplates, wherein the mixture is incubated and then centrifuged. Various reactions then occur or do not occur depending on, for example, the blood types of the red blood cells or whether certain antibodies are present within the blood sample. These reactions manifest themselves as clumps of cells or as particles with antigens or antibodies on their surfaces, referred to as agglutinates. The failure of any agglutinates to appear indicates no reaction has occurred, while the presence of agglutinates, depending on the size and amount of the clumps formed, indicates the presence of a reaction and the level of concentration in the sample and reaction strength.

Rather than using microplates or test tubes, another form of agglutination test method has been more recently utilized, as is described in U.S. Patent No. 5,512,432 to LaPierre et al. According to this method, gel or glass bead microparticles are contained within a small column, referred to as a microcolumn or a microtube. A reagent, such as antibody for detecting "A" antigen, is dispensed in a diluent in the microcolumn and test red blood cells, which may or may not contain "A" antigen, are placed in the reaction chamber above the column. The column, which is typically one of a plurality of columns formed in a transparent card or cassette, is then centrifuged. The centrifugation accelerates the reaction, if any, between the red blood cells and the reagent, and also urges any cells toward the bottom of the column. In the meantime, the glass beads or the gel material acts as a filter, and resists or impedes downward movement of the particles in the column. As a result, the nature and distribution of the particles in the microcolumn provides a visual indication of whether any agglutination reaction has occurred, and if such a reaction has occurred, the strength of the reaction based on the relative position of the agglutinates in the column.

If no agglutination reaction has occurred, then all or virtually all of the red blood cells in the column will pass downward during the centrifugation procedure, to the bottom of the column in the form of a pellet. Conversely and if there is a strong reaction between the reagent and the red blood cells, then virtually all of the red blood cells will agglutinate, and large groupings will form at the top of the microtube above the gel or bead matrix in that the matrix is sized not to let these clumps pass through. Reactions falling between these latter two extremes are possible in which some but not all of the red blood cells will have agglutinated. The percentage of red blood cells that agglutinate and the size of the agglutinated particles each have a relationship with the strength of the reaction. Following the centrifugation process and after all processing steps have been completed, the microtube is visually examined by either a human operator or by machine vision and the reaction between the red blood cells and the reagent is then classified. The reaction is classified as being either positive or negative, and if positive, the reaction is further typically classified into one of four classes depending on the strength of the reaction.

Automated immunodiagnostic testing apparatus or systems have been designed that are used for the handling, testing and evaluation of "gel cards", "bead cassettes" or other forms of test elements such as described above that employ column agglutination technology. In a typical automated apparatus, a control module or station having at least one imager and a connected processor is used to evaluate the results of testing following the completion of a centrifugation cycle, this cycle typically lasting between about 10 and 30 minutes. Following centrifugation in conventionally known apparatus, the test elements must first be removed from the centrifuge and then relocated within the control station of the apparatus or the test element can be removed from the centrifuge and evaluated manually to determine the extent of the agglutination reaction, if any.

A general and continuing problem in the field of immunodiagnostic testing is that of improving throughput and processing time, particularly with automated analysis systems or apparatus. In addition and for similar reasons, it is desirable to terminate testing if, for example, a failure mode occurs that would produce an obviously incorrect result and waste considerable time, if centrifugation were to proceed over its complete typical time cycle. It is believed that there are methods to determine or predict certain test results (e.g., strong positive or strong negative reactions) in advance of a complete centrifugation cycle.

EP1526370 teaches an apparatus for classifying a liquid patient sample which includes at least one sample container having a quantity of a sample that is aggressively acted upon so as to create a flow field.

JP03110468 teaches an apparatus which allows the detecting of agglutination patterns while a circular microplate is being turned.

EP1450159 teaches an apparatus for the detection of agglutination of assays.

US4297104 (A) relates to a method for detecting or identifying virus antigens or erythrocyte or cell antigens or antibodies in a biological medium.

### SUMMARY OF THE INVENTION

According to one aspect, there is disclosed an immunodiagnostic testing apparatus comprising:
a housing;
a controller (280);
a test element (60) which comprises at least one of a gel card or a bead cassette;
a centrifuge (224) disposed within said housing, said centrifuge including a rotatable arm member (228) having a pair of opposing ends (232, 236) that extend radially outward from a center hub (240) wherein each of the ends (232, 236) of the rotatable arm member (228) is configured to support at least one test element (60) thereupon for centrifugation over a predetermined time period as controlled by said controller (280);
an imaging assembly (250) disposed in proximity to said centrifuge (224) such that at least one image can be captured of at least one test element (60) prior to the conclusion of said predetermined centrifugation time period wherein said test element is capable of producing a perceivable agglutination reaction that can be graded, said reaction being accelerated by centrifugation ; and
an illumination source (272) disposed in relation to said imaging assembly, wherein said imaging assembly (250) and said illumination source are synchronized by said controller (280) with said centrifuge (224) to provide in situ images of said at least one test element (60);
in which said at least one captured image of said test element taken prior to the conclusion of said predetermined centrifugation time period includes predictive data, said predictive data being indicative of whether testing can be stopped in advance of the conclusion of the centrifugation time period and wherein the controller 280 is configured to stop the motor 244 of the centrifuge 224 when the grade of the agglutination reaction is predictive.

The test element is capable of producing a perceivable agglutination reaction that can be graded, wherein the agglutination reaction is being accelerated by centrifugation and wherein at least one captured image of said test element is taken prior to the conclusion of a predetermined centrifugation period and used by means of the apparatus as to the processing of at least one test element.

In one version, the imager can capture a single image or multiple images of the at least one test element during the course of a centrifugation process such that a predicted value of a gradable agglutination reaction can be obtained. If adequate predictive data can be obtained, the centrifugation process can be stopped before the completion time of a typical centrifugation period. In one version, a rate of change in the reaction can be detected by inspection of captured images, either in sequence or in comparison to a standard image. For example and according to one version, a rate of change can determined whether on the basis of distance traveled over time to predict an endpoint of the reaction. Alternatively, the at least one captured image can be used to determine whether a failure mode exists, for example, either in the test element or in the process itself. An illumination source is provided that can be activated for the purposes of imaging, such as a strobe lamp or other controllable light source. In one version, a strobe lamp and an imager are each synchronized with the rotation of the proximity therewith.

The imager operates dynamically; that is. "on the flv". to provide data while the centrifuge is still in operation.

There is disclosed a method for performing immunodiagnostic testing, said method including the steps of: adding patient sample to a test element for purposes of creating an agglutination reaction that can be graded, said test element including at least one column retaining an inert test material, a reagent, and a quantity of said patient sample wherein the test element is capable of producing an agglutination reaction; placing said test element within a centrifuge; centrifuging said test element to accelerate the agglutination reaction; and imaging said test element prior to the completion of a full centrifugation cycle to obtain predictive test data.

The method further includes the steps of predicting the grade of a agglutination reaction formed within the test element prior to the termination of the centrifugation cycle and terminating the centrifuging step based on predictive data obtained. According to this method, either single and/or multiple in-situ images can be obtained in which the imaging step can be performed dynamically while the centrifuge is still operating or statically at an intermediate point in the test cycle with the test element either in the centrifuge or separately removed for evaluation.

According to another version, there is described an immunodiagnostic testing apparatus comprising a centrifuge, at least one imager disposed in proximity to said centrifuge such that at least one image can be captured of at least one test element that is capable of producing a perceivable agglutination reaction that can be graded. The at least one imager is disposed to capture said at least one image while said at least one test element is in the centrifuge and in which said at least one captured image is captured prior to the conclusion of a predetermined centrifugation time period.

One advantage realized using the apparatus and methods as described herein is that it is possible to extract more data concerning aspects of an immunodiagnostic test and also determine an earlier result than with currently known testing apparatus. It is also advantageous in that failure modes can also be identified at an earlier point in the process, thereby providing significant time savings and providing additional scheduling opportunities.

In addition, providing in-situ imaging of the test elements provides drastic improvements to time management using testing apparatus in that immunodiagnostic test elements no longer require a separate operation to transport them into a reader queue, thereby simplifying and optimizing the design and footprint of such apparatus, while also significantly improving throughput.

These and other features and advantages will become readily apparent from the following Detailed Description, which should be read in conjunction with the accompanying drawings.

### BRIEF DESCRIPTION OF THE DRAWINGS

Fig. 1 is a simplified top plan view of a prior art automated blood analysis apparatus;
Fig. 2 is a simplified front view of the automated blood analysis apparatus of Fig. 1;
Figs. 3 and 4 are front views of a prior art immunodiagnostic test element, in which Fig. 3 depicts the test element prior to testing and Fig. 4 depicts the test element following testing and after agglutination reactions have occurred within the columns of the test element;
Fig. 5 depicts a partial view of a portion of an automated apparatus that includes an integrated centrifugation/imaging system;
Fig. 6 is a schematic block diagram of an automated immunodiagnostic test apparatus that includes the integrated system of Fig. 5; and
Fig. 7 is a flow chart of a test procedure employing the apparatus of Figs. 5 and 6.

### DETAILED DESCRIPTION

The following relates to apparatus and related methods for immunodiagnostic testing of at least one exemplary test element, in this example, a "gel card" or "bead cassette". It will be readily apparent that other forms of apparatus as well as other forms of test elements, such as, microplates and the like can be incorporated that apply the same inventive aspects. In addition and in order to provide a suitable frame of reference with regard to the accompanying drawings, certain terms are used throughout. It is not intended that these terms are limiting of the scope of the inventive concepts described herein, except in instances where so specifically indicated.

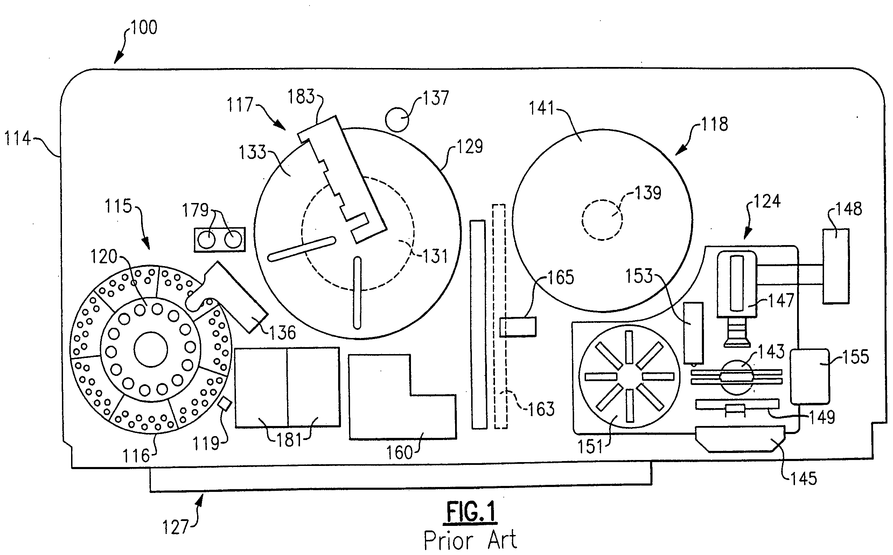

Referring to Figs. 1-2, there is shown a prior art immunodiagnostic testing apparatus. According to this specific example, the apparatus is an AutoVue® diagnostic apparatus manufactured by Ortho-Clinical Diagnostics, Inc, which is generally labeled herein as reference numeral 100. This testing apparatus 100, as described in greater detail in commonly-assigned U.S. Patent 5,578,269 to Yaremko et al., is defined generally by a frame 114 that houses a plurality of individual modules or assemblies, including a sample and reagent holding supply 115, an incubator station 117, a centrifuge 118, an analysis station 124 and a drawer assembly 127, each shown in Fig. 1.

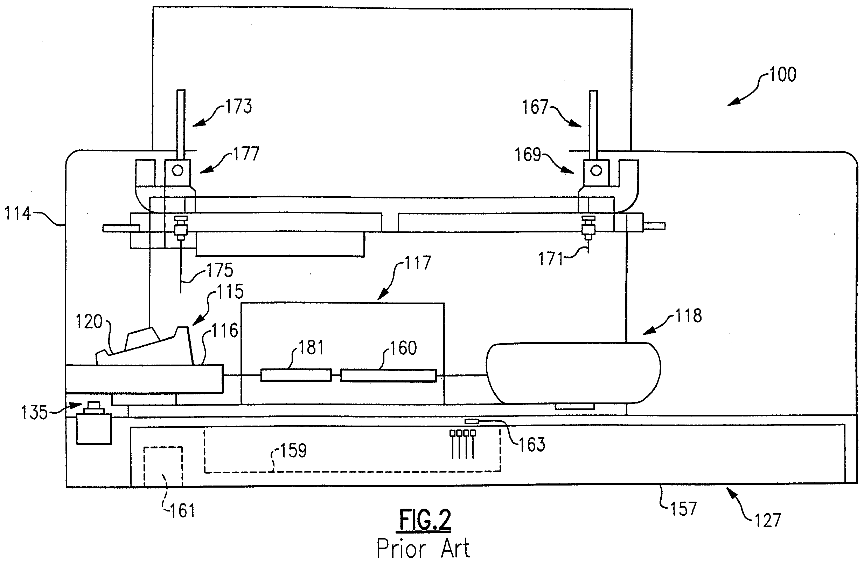

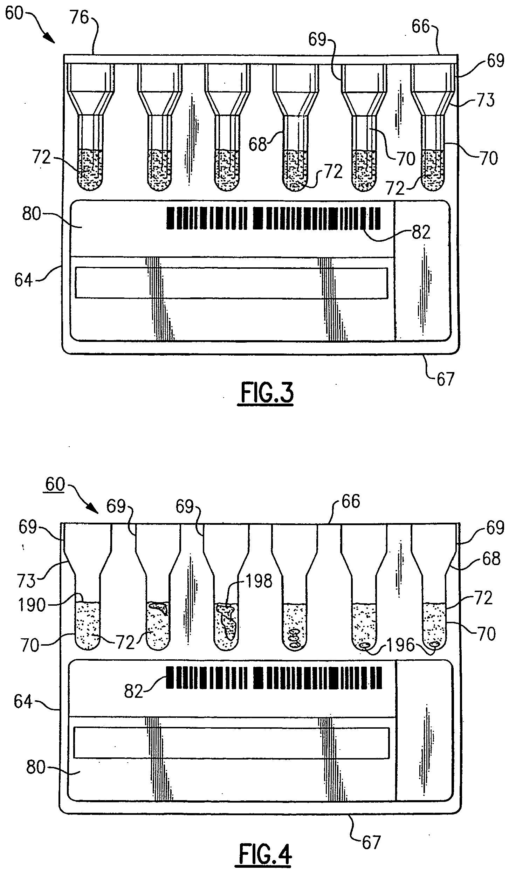

More particularly, the sample and reagent holding supply 115 according to this specific apparatus 100 includes a plurality of patient vials that are disposed in a sample rack 116, as well as reagents that are separately disposed within vials maintained within a reagent rack 120. A bar code reader 119 is also provided to identify the sample being tested wherein the vials include labels (not shown) with encoded symbology such as the lot number, expiration date and other pertinent information. A drive mechanism 135, shown in Fig. 2, is used to rotate the supply wherein a tube hold-down apparatus 136 is further included. The incubator station 117 includes a cassette rack 129 that further includes respective first and second stations 131, 133, as well as a drive mechanism that includes a motor 137. The centrifuge 118 includes a motor 139 and a rotor 141. The analysis station 124 includes holding means 143, illumination assembly 145, an imaging subsystem 147, a processing subsystem 148 connected to the imaging subsystem, a transport subsystem 149, a storage rack 151, a bar code reader 153, and a waste receptacle 155. Referring to Figs. 1 and 2, the drawer assembly 127 includes a drawer 157, a slide tray 159, a holding area 160, a motor 161, a sensor bar 163 and a bar code reader 165. A transport assembly 167 of the apparatus 100 includes a robot arm 169 and a gripper 171. A pipette assembly 173 includes a pipette 175 attached to a robot arm 177, this assembly further including shallow and deep wash areas 179, as well as cell dilution packs 181.
Before describing the operation of the apparatus 100 and referring to Fig. 3, there is shown an exemplary test element 60 that can be used with the automated immunodiagnostic testing apparatus 100 of Figs. 1-2. This test element 60 is defined by a planar substrate 64 made from plastic or other suitable material that includes a top side 66 and an opposing bottom side 67. A plurality of transparent microtubes 68 are supported by the planar substrate 64, each of the microtubes being vertically arranged and spaced from one another. Each microtube 68, as provided in the herein described test element 60, includes an upper portion 69 having an open end which is coplanarly arranged with the top side 66 of the element. Each upper portion 69 extends into a lower portion 70, the lower portion having a smaller diameter than that of the upper portion and in which the upper and lower portions are separated by a transitional inwardly tapering portion 73. Each microtube 68 contains a quantity of an inert material 72, such as a matrix of gel particles or a plurality of glass beads, that may be coated with an antigen or antibody to form an aqueous medium or suspension. A pierceable foil wrap 76 is secured to the top side 66 of the test element 60 that covers and closes the tops of each of the microtubes 68, sealing same and wherein the antigens or antibodies have been added prior to the addition of the foil seal at the time of manufacture. Each test element 60 further includes a label 80 as well as a bar code 82, each identifying various data regarding the element, including the test element type, date of manufacture, and recommended expiration date of the test element and its contents. This bar code 82 may include other data, such as the test element manufacturer, as well as the time and place of manufacture, lot number and other information.

Referring to Figs. 1-3 and as to the general operation of the testing apparatus 100, a plurality of test elements 60 are initially loaded into the testing apparatus through the drawer assembly 127 to a position in which the gripper 171 of the transport assembly 167 has access. A test element 60 is then picked up by the gripper 171 and is moved to the bar code reader 165 of the drawer assembly 127 wherein the label information of the test element is read and verified. Following reading, the test elements 60 are loaded into the incubator 117 by means of the gripper 171, and more specifically the cassette rack 129, in relation to a piercing assembly 183. The piercing assembly 183 of the herein described testing apparatus 100 is used to pierce openings in the foil wrap 76 of the test element 60 by means of a plurality of piercing elements (not shown) disposed on a rotating assembly (not shown). Alternatively, the pipette 175 or other piercing means could be utilized for this function. The piercing elements are driven by means of solenoids or similar means in reciprocating fashion. The incubator 117 then moves the test element 60 into a position that enables the pipette 175 to dispense a quantity of patient sample into the columns. The pipette 175 is operated to draw fluids from the reagent and sample racks 116, 120 of the sample and reagent supply 115. Upon piercing or removing the foil wrap 76, a predetermined quantity of patient sample (in the form of red blood cells (RBCs) and sera) from the sample supply 115 is added to the lower portion 70 of each microtube 68 via the pipette assembly 127 and the test element 60 is then incubated. The test element 60 can then be gripped and transported to the centrifuge 118 by means of the gripper 171 of the transport assembly 167. Subsequently, the test element is spun down in order according to a predetermined protocol in order to accelerate an agglutination reaction (if any) between the reagent and sample that can be graded, for example, for blood grouping.

In terms of processing, a typical centrifugation cycle may extend for 10-20 minutes or more, depending on the nature of the specific test that is being performed by the apparatus. In order to evaluate the resulting reaction, if any, the test elements 60 are then removed from the centrifuge 118 of the testing apparatus 100 by means of the gripper 171 and the test element is transferred to the storage rack 151 of the analysis station 124. This rack 151 then rotates in order to position the test element 60 immediately adjacent the test element holder 143. The transport subsystem 149 of the analysis station 124 subsequently transfers the test element 60 from the storage rack 151 to the element holder 143 and a digitized image of the test element, or pertinent portions thereof, is obtained using the imaging subsystem 147 in combination with the illumination assembly 145. The digitized data of the test element 60 is then utilized by the connected processing subsystem 148 in order to determine if a reaction has occurred in the test element and if so, the classification of the reaction. This determination can be also be made based on the images using machine vision.

An exemplary test element 60 is shown in Fig. 4 following centrifugation, and in which varying reactions have occurred in the supported microtubes 68 thereof. The principle of the microtubes 68 of the test element 60 is that the reagent coated upon the inert material 72 can react with the red blood cells of the patient sample and form clumps (agglutinates) in which filtering prevents large clumps indicative of a strongly positive reaction to pass therethrough. In this instance, a layer of agglutinated particles 190 would form above the gel or bead matrix, providing a visual indication of the strongly positive reaction. Conversely and in the absence of any reaction, a pellet of red blood cells 196 would pass through the inert material 72 and settle at the bottom of the microtube 68, indicating a negative reaction. Varying grades of reaction between these two extremes may also be possible wherein agglutinates 198 are disposed throughout the column. Alternatively, the test elements 60 can be visually inspected upon removal from the centrifuge 118.

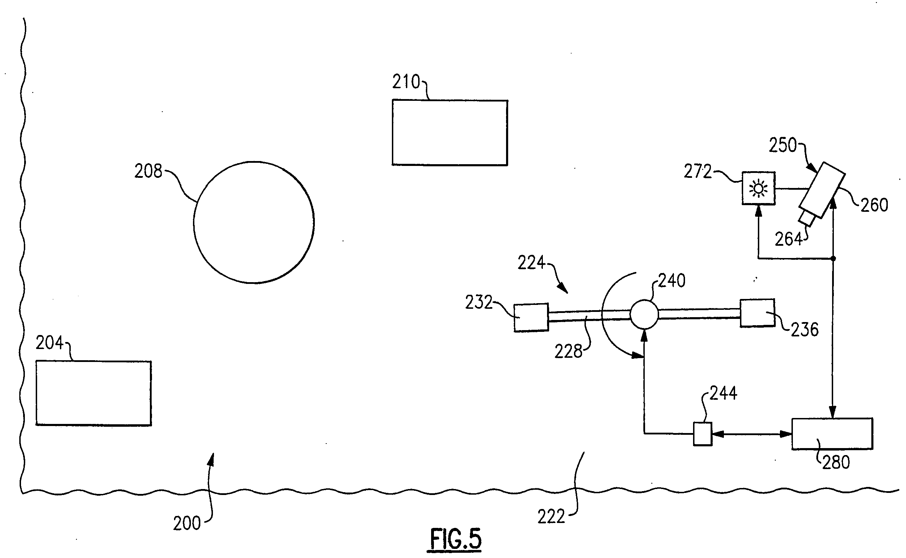

With the foregoing serving as background and referring to Fig. 5, there is shown a centrifuge chamber or module 222 of a testing apparatus 200 in accordance with one embodiment of the invention. The herein described testing apparatus 200 is merely shown schematically and includes a patient and reagent supply 204, an incubator 208 and a transport assembly 210.

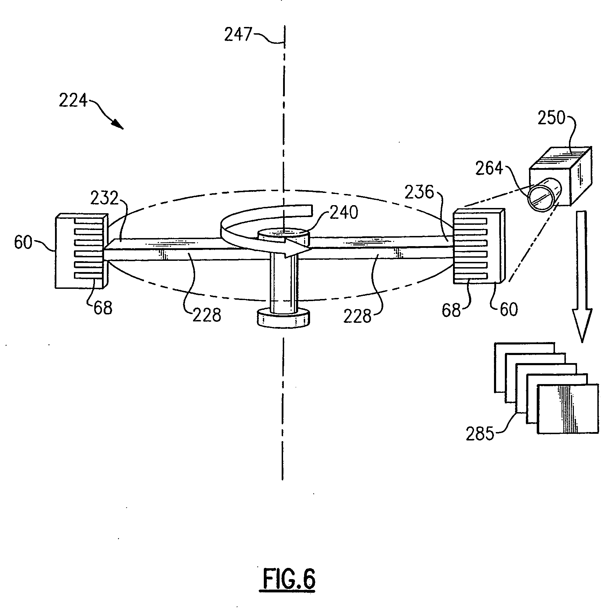

The centrifuge chamber or module 222 of the herein described apparatus is defined by a housing (not shown) that retains a centrifuge 224. The centrifuge 224 according to this embodiment includes a rotatable arm member 228 having a pair of opposing ends 232, 236 that extend radially outward from a center hub 240. Each of the ends 232, 236 of the rotatable arm member 228 are configured to support at least one test element 60 thereupon, such as the exemplary element described above with regard to Fig. 3. According to the present embodiment, a test element 60 is supported by a clamp at each arm end 232, 236 with the top end 66, Fig. 3, of the element being supported such that the plurality of microtubes 68 are arranged horizontally, as shown in Fig. 6.

Referring to Figs. 5 and 6, the rotatable arm member 228 is fixedly secured to the center hub 240, wherein the hub and therefore the arm member are each caused to rotate about a vertically disposed axis 247 by means of a drive mechanism including a motor, shown herein schematically in Fig. 5 by reference numeral 244.

According to a preferred version, an imaging assembly 250 is disposed within the centrifuge chamber 222 of testing apparatus 220 is preferably mounted in a fixed location within the housing such that the ends 232, 236 of the rotatable arm member 228 of the centrifuge 224 pass in relation thereto. It should be noted in passing that alternative mounting schemes can be provided. For example, a bucket-type centrifuge can be provided in which a portion of the centrifuge supporting the at least one test element can pivot outwardly away from a center hub under the influence of centrifugal force. In this instance, the imaging assembly can be alternatively positioned so as to access the at least one test element from above. Other similar configurations are possible.

The imaging assembly 250 according to this embodiment includes at least one electronic imager, such as a CCD or CMOS-type imager having an array of pixels that are disposed within a housing 260 having an opening 264 that permits an image of the rotatable arm member 228, and in particular a retained test element 60, to be captured. Alternatively, other imaging means, such as a conventional camera, can be utilized. An illumination assembly 2 72 is disposed in relation to the imaging assembly 250 and includes a light source such as a strobe lamp, at least one LED, incandescent lamp, or other suitable source capable of emitting light. The illumination assembly can be separately disposed in relation to the imager assembly 250 or can be integrated directly therein, such as a flash assembly of a conventional 35 mm camera, by way of example.

Referring to Fig. 5 and according to one version, control of the imaging assembly 250 and the illumination assembly 272 is synchronized by a controller, shown schematically herein as 280, with the rotational position of the arm member 228 of the centrifuge 224 to capture at least one image of the test element 60 during the centrifugation process at an intermediate point during the cycle. Multiple images or a sequence of images, shown schematically as 285 in Fig. 6) can therefore be obtained in-situ by suitable control of the integration time of the electronic imager in conjunction with the rotational speed of the centrifuge 224. The resulting images are transmitted to the controller 280, which according to this embodiment includes processing means for providing an in-situ digitized image. Using at least one in-situ image, it is possible to provide predictive gradations of a resulting agglutination reaction (or lack of reaction) to the user of the apparatus 200. In another version, the rate of change of agglutinates traveling through the inert matrix can be determined algorithmically based upon captured advance of a complete centrifugation cycle. Alternatively, the image results can be directed to the user for display in situ wherein the user can elect to terminate the centrifugation process in advance of a completed cycle. As such, processing time for immunodiagnostic tests can be effectively and significantly reduced.

Also disclosed is an alternative version in which, rather than using the imager "on the fly". the centrifuge 222 can be stopped at an intermediate point in the cycle and the imaging assembly 250 can be used to capture at least one image of the test element 60, and particularly that of the transparent microtubes 68. Alternatively, the centrifuge 222 can be stopped and the at least one supported test element 60 can be removed by means of a gripper or other means using a transfer assembly of the apparatus or otherwise at a predetermined intermediate point in the centrifugation time cycle prior to completion. The test element(s) can then be transferred to the analysis station of the testing apparatus, such as that previously described with regard to Figs 2 and 3, and at least one image of at least one column can be captured for evaluation using a contained imaging assembly. The resulting image(s), like those of the preceding, can be used to make a predictive conclusion of the grade of the resulting reaction, if any, or to identify a failure mode of the process or a test element in advance of a complete centrifugation cycle. It should be further pointed out that the form of centrifuge employed is not necessarily critical to the workings of the invention provided that the imager, if used within the centrifuge module, can adequately access a retained test element(s) and therefore the one that is described is intended to be merely exemplary.

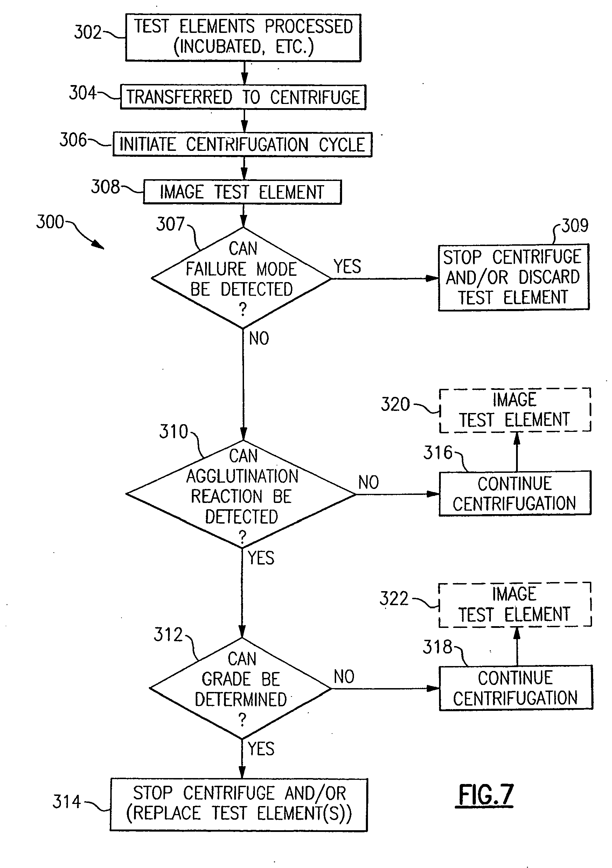

Referring to Fig. 7, there is shown a flow chart 300 depicting a process methodology employing the configurations shown in Figs. 5 and 6, and for purposes of the description using test elements 60, Figs. 3 and 4, or other elements that are capable of producing a visually perceivable agglutination reaction. Following the flow chart of Fig. 7, but referring to the remaining figures and according to step 304, test elements 60 are loaded into the centrifuge 222 by way of the transport assembly 210 following incubation and earlier processing steps, as described in the preceding, step 302. Preceding this loading and though not indicated on the flow chart, a first image might be captured of the test element 60 either in the analysis station or otherwise, in order to provide a standard image prior to loading of patient sample or prior to loading into the centrifuge 222. According to one version, the centrifuge 222 is typically programmed to run for a predetermined period or cycle (e.g., 10-30 minutes), which is initiated per step 306, using the controller 280. According to this version, however, the imaging assembly 250 is utilized to capture in-situ images of the test element(s) 60 during the centrifugation cycle, whether by terminating the centrifuge and capturing an image, terminating the centrifuge and removing the test element to obtain an image or by capturing at least one image dynamically, or on the fly, based on the position of the rotating arm member 228, step 310, and to determine based on the results of the at least one captured image whether there is sufficient information to predict in advance whether a reaction has occurred, step 310, and if so, the grade of the reaction, step 312, (i.e., strongly positive or strongly negative) or whether a failure mode has occurred, step 313. In the case in which a standard image is first captured and reveals, for example, a defect (e.g., a scratch) on the test element, this feature can be subtracted from resulting images so as not bias the result.

Essential to the teachings described herein and in either instance; that is, whether the imager assembly is located within the centrifuge for activation of the imaging assembly 250 or an image is captured statically within or in another part of the testing apparatus, the at least one image is captured at an intermediate point in the centrifugation cycle, but prior to conclusion thereof. If the results indicate that a predictive grade of the reaction (for example, no reaction or strong reaction) can be made or that a failure mode exists then, according to step 314, the controller 280 either causes the motor 244 to stop the centrifuge 222 in advance of the completion of the centrifugation cycle and the test element 60 is removed in the case of an embedded imager or the test element is not placed back into the centrifuge in the instance in which a test element is first removed prior to capturing the at least one image. If a predictive grade cannot be made or if a failure mode cannot be identified, then centrifugation proceeds, step 316, wherein another image, steps 320, 322 may be taken in advance of completion of the cycle time and the foregoing steps are repeated. In one version, multiple images can be taken intermediately in the centrifugation cycle so as to determine the rate of change of the reaction (i.e., movement of agglutinates in distance as measured over time), if any, and of process endpoint, for example by means of a velocity vector of the agglutinates through the inert matrix of the column as determined algorithmically based on the captured images. In the instance in which the at least one image is taken outside of the centrifuge, the test element is routed back to the centrifuge by the transport assembly of the apparatus and the centrifugation cycle resumes.

## Claims

1. An immunodiagnostic testing apparatus, said apparatus comprising:
a housing;
a controller (280);
a test element (60) which comprises at least one of a gel card or a bead cassette;
a centrifuge (224) disposed within said housing, said centrifuge including a rotatable arm member (228) having a pair of opposing ends (232, 236) that extend radially outward from a center hub (240) wherein each of the ends (232, 236) of the rotatable arm member (228) is configured to support at least one test element (60) thereupon for centrifugation over a predetermined time period as controlled by said controller (280);
an imaging assembly (250) disposed in proximity to said centrifuge (224) such that at least one image can be captured of at least one test element (60) prior to the conclusion of said predetermined centrifugation time period wherein said test element is capable of producing a perceivable agglutination reaction that can be graded, said reaction being accelerated by centrifugation ; and
an illumination source (272) disposed in relation to said imaging assembly, wherein said imaging assembly (250) and said illumination source are synchronized by said controller (280) with said centrifuge (224) to provide in situ images of said at least one test element (60);
in which said at least one captured image of said test element taken prior to the conclusion of said predetermined centrifugation time period includes predictive data, said predictive data being indicative of whether testing can be stopped in advance of the conclusion of the centrifugation time period and wherein the controller 280 is configured to stop the motor 244 of the centrifuge 224 when the grade of the agglutination reaction is predictive.

2. A testing apparatus as recited in Claim 1, wherein said at least one illumination source is a strobe lamp.

3. A testing apparatus as recited in Claim 1, wherein said imaging assembly (250) is held in fixed relation to said centrifuge (224).

4. A testing apparatus as recited in Claim 1, wherein said imaging assembly (250) includes an electronic imager.

5. A testing apparatus as recited in Claim 1, wherein the test element comprises a plurality of transparent microtubes.

6. A method for predicting the extent of an agglutination reaction of at least one test element (60) in a testing apparatus, said at least one test element being capable of producing a perceivable agglutination reaction, said testing apparatus including a centrifuge (224) and a controller (280) configured to stop the motor (244) of the centrifuge (224) when the grade of the agglutination reaction is predictive, said method comprising the steps of:
placing at least one test element (60) capable of producing a perceivable agglutination reaction within said centrifuge (224), wherein the test element (60) comprises at least one of a gel card or a bead cassette;
centrifuging said test elements (60) to accelerate said agglutination reaction;
imaging said at least one test element (60) at an intermediate point in the centrifugation cycle, but prior to conclusion thereof, wherein said imaging step occurs dynamically by capturing at least one image of said test element while said centrifuge (224) is being operated;
stopping said centrifuging step in advance of a predetermined time period based on predictive data obtained from images obtained from said imaging step; and
removing at least one test element (60) whose predictive data is sufficient to predict the outcome of the reaction.

7. A method as recited in Claim 6, including the step of adding patient sample to said test element prior to said placing step, said test element including at least one column (66) retaining an inert test material and a reagent capable of producing a perceivable agglutination reaction with said sample.

8. A method as recited in Claim 6, wherein said predictive data is indicative of at least one failure mode of said apparatus.

9. A method as recited in Claim 6, wherein the test element comprises a plurality of transparent microtubes.

## Patentansprüche

1. Immundiagnostische Testvorrichtung, wobei die Vorrichtung Folgendes umfasst:
ein Gehäuse;
eine Steuereinheit (280);
ein Testelement (60), das mindestens eines aus einer Gelkarte oder einer Kugelkassette umfasst;
eine Zentrifuge (224), die in diesem Gehäuse angeordnet ist, wobei diese Zentrifuge ein drehbares Armelement (228) einschließt, das ein Paar gegenüberliegender Enden (232, 236) aufweist, die sich von einer zentralen Nabe (240) nach außen erstrecken, wobei jedes der Enden (232, 236) des drehbaren Armelements (228) konfiguriert ist, um mindestens ein Testelement (60) darauf zum Zentrifugieren über eine bestimmte Dauer, die von der Steuereinheit (280) gesteuert wird, zu tragen;
eine Bilddarstellungsbaugruppe (250), die in der Nähe der Zentrifuge (224) angeordnet ist, so dass mindestens ein Bild von mindestens einem Testelement (60) vor Abschluss der vorbestimmten Zentrifugierdauer aufgenommen werden kann, wobei das Testelement in der Lage ist, eine merkliche Agglutinationsreaktion zu erzeugen, die in Grade eingeteilt werden kann, wobei die Reaktion durch Zentrifugieren beschleunigt wird; und
eine Lichtquelle (272), die in Bezug auf die Bilddarstellungsbaugruppe angeordnet ist, wobei die Bilddarstellungsbaugruppe (250) und die Lichtquelle durch die Steuereinheit (280) mit der Zentrifuge (224) synchronisiert werden, um in situ Bilder von dem mindestens einen Testelement (60) bereitzustellen;
wobei das mindestens eine aufgenommene Bild des Testelements, das vor Abschluss der vorbestimmten Zentrifugierdauer erstellt wird, prognostische Daten einschließt, wobei die prognostischen Daten darauf hindeuten, ob der Test vor Abschluss der Zentrifugierdauer gestoppt werden kann, und wobei die Steuereinheit 280 konfiguriert ist, um den Motor 244 der Zentrifuge 224 zu stoppen, wenn der Grad der Agglutinationsreaktion vorhersehbar ist.

2. Testvorrichtung nach Anspruch 1, wobei die mindestens eine Lichtquelle eine Stroboskoplampe ist.

3. Testvorrichtung nach Anspruch 1, wobei die Bilddarstellungsbaugruppe (250) in Bezug auf die Zentrifuge (224) fixiert gehalten wird.

4. Testvorrichtung nach Anspruch 1, wobei die Bilddarstellungsbaugruppe (250) ein elektronisches Bilddarstellungsgerät einschließt.

5. Testvorrichtung nach Anspruch 1, wobei das Testelement eine Vielzahl transparenter Mikroröhrchen umfasst.

6. Verfahren zur Prognose des Umfangs einer Agglutinationsreaktion von mindestens einem Testelement (60) in einer Testvorrichtung, wobei das mindestens eine Testelement in der Lage ist, eine merkliche Agglutinationsreaktion zu erzeugen, wobei die Testvorrichtung eine Zentrifuge (224) und eine Steuereinheit (280) einschließt, die konfiguriert ist, um den Motor (244) der Zentrifuge zu stoppen, wenn der Grad der Agglutinationsreaktion vorhersehbar ist, wobei das Verfahren die folgenden Schritte umfasst:
Platzieren mindestens eines Testelements (60), das in der Lage ist, eine merkliche Agglutinationsreaktion zu erzeugen, in der Zentrifuge (224), wobei das Testelement (60) mindestens eines einer Gelkarte oder einer Kugelkassette umfasst;
Zentrifugieren der Testelemente (60), um die Agglutinationsreaktion zu beschleunigen;
bildliche Darstellung des mindestens einen Testelements (60) an einem Zwischenpunkt im Zentrifugierzyklus, jedoch vor dessen Abschluss, wobei der Schritt der bildlichen Darstellung dynamisch durch Aufnehmen mindestens eines Bildes des Testelements auftritt, während die Zentrifuge (224) in Betrieb ist;
Stoppen des Zentrifugierschritts vor Ablauf einer vorbestimmten Dauer, basierend auf prognostischen Daten, die von Bildern erhalten werden, die in dem Schritt der bildlichen Darstellung erhalten werden; und
Entfernen mindestens eines Testelements (60), dessen prognostische Daten ausreichend sind, um das Ergebnis der Reaktion vorherzusagen.

7. Verfahren nach Anspruch 6, den Schritt des Zugebens einer Patientenprobe zu dem Testelement vor dem Schritt des Platzierens einschließend, wobei das Testelement mindestens eine Säule (66) einschließt, die ein inertes Testmaterial enthält, und ein Reagens, das in der Lage ist, eine merkliche Agglutinationsreaktion mit dieser Probe zu erzeugen.

8. Verfahren nach Anspruch 6, wobei die prognostischen Daten auf mindestens eine Ausfallart der Vorrichtung hindeuten können.

9. Verfahren nach Anspruch 6, wobei das Testelement eine Vielzahl transparenter Mikroröhrchen umfasst.

## Revendications

1. Appareil d'évaluation d'immunodiagnostic, ledit appareil comprenant :
un boîtier ;
un dispositif de commande (280) ;
un élément d'évaluation (60) qui comprend au moins l'une d'une carte gel ou d'une cassette de billes ;
une centrifugeuse (224) disposée au sein dudit boîtier, ladite centrifugeuse comportant un organe de bras rotatif (228) ayant une paire d'extrémités opposées (232, 236) qui s'étendent radialement vers l'extérieur depuis un moyeu central (240) dans lequel chacune des extrémités (232, 236) de l'organe de bras rotatif (228) est configurée pour supporter au moins un élément d'évaluation (60) sur elle pour centrifugation sur une période prédéterminée commandée par ledit dispositif de commande (280) ;
un ensemble d'imagerie (250) disposé à proximité de ladite centrifugeuse (224) de sorte qu'au moins une image puisse être capturée d'au moins un élément d'évaluation (60) avant la conclusion de ladite période de centrifugation prédéterminée dans lequel ledit élément d'évaluation est capable de produire une réaction d'agglutination perceptible qui peut être classée, ladite réaction étant accélérée par centrifugation ; et
une source d'illumination (272) disposée par rapport audit ensemble d'imagerie, dans lequel ledit ensemble d'imagerie (250) et ladite source d'illumination sont synchronisés par ledit dispositif de commande (280) avec ladite centrifugeuse (224) pour fournir des images *in situ* dudit au moins un élément d'évaluation (60) ;
dans lequel ladite au moins une image capturée dudit élément d'évaluation prise avant la conclusion de ladite période de centrifugation prédéterminée comporte des données prédictives, lesdites données prédictives étant indicatives du fait que l'évaluation peut être arrêtée ou non avant la conclusion de la période de centrifugation et dans lequel le dispositif de commande (280) est configuré pour arrêter le moteur 244 de la centrifugeuse 224 lorsque la classification de la réaction d'agglutination est prédictive.

2. Appareil d'évaluation selon la revendication 1, dans lequel ladite au moins une source d'illumination est une lampe stroboscopique.

3. Appareil d'évaluation selon la revendication 1, dans lequel ledit ensemble d'imagerie (250) est maintenu en relation fixée à ladite centrifugeuse (224).

4. Appareil d'évaluation selon la revendication 1, dans lequel ledit ensemble d'imagerie (250) comporte un imageur électronique.

5. Appareil d'évaluation selon la revendication 1, dans lequel l'élément d'évaluation comprend une pluralité de microtubes transparents.

6. Procédé de prédiction de l'étendue d'une réaction d'agglutination d'au moins un élément d'évaluation (60) dans un appareil d'évaluation, ledit au moins un élément d'évaluation étant capable de produire une réaction d'agglutination perceptible, ledit appareil d'évaluation comportant une centrifugeuse (224) et un dispositif de commande (280) configuré pour arrêter le moteur (244) de la centrifugeuse (224) lorsque la classification de la réaction d'agglutination est prédictive, ledit procédé comprenant les étapes de :
placement d'au moins un élément d'évaluation (60) capable de produire une réaction d'agglutination perceptible au sein de ladite centrifugeuse (224), dans lequel l'élément d'évaluation (60) comprend au moins l'une d'une carte gel ou d'une cassette de billes ;
centrifugation desdits éléments d'évaluation (60) pour accélérer ladite réaction d'agglutination ;
imagerie dudit au moins un élément d'évaluation (60) à un point intermédiaire dans le cycle de centrifugation, mais avant sa conclusion, dans lequel ladite étape d'imagerie se produit dynamiquement par la capture d'au moins une image dudit élément d'évaluation tandis que la centrifugeuse (224) est en fonctionnement ;
arrêt de ladite étape de centrifugation avant une période prédéterminée d'après des données prédictives obtenues à partir d'images obtenues par ladite étape d'imagerie ; et
élimination d'au moins un élément d'évaluation (60) dont les données prédictives sont suffisantes pour prédire l'issue de la réaction.

7. Procédé selon la revendication 6, comportant l'étape d'ajout d'un échantillon de patient audit élément d'évaluation avant ladite étape de placement, ledit élément d'évaluation comportant au moins une colonne (66) retenant un matériau d'évaluation inerte et un réactif capable de produire une réaction d'agglutination perceptible avec ledit échantillon.

8. Procédé selon la revendication 6, dans lequel lesdites données prédictives sont indicatives d'au moins un mode de défaillance dudit appareil.

9. Procédé selon la revendication 6, dans lequel l'élément d'évaluation comprend une pluralité de microtubes transparents.
